## Europäisches Patentamt
## European Patent Office
## Office européen des brevets

(19)

(11) Veröffentlichungsnummer: **0 217 191**
**B1**

(12) **EUROPÄISCHE PATENTSCHRIFT**

(45) Veröffentlichungstag der Patentschrift:
**22.06.88**

(21) Anmeldenummer: **86112545.8**

(22) Anmeldetag: **10.09.86**

(51) Int. Cl.⁴: **C 07 C 53/12,** C 07 C 53/08,
C 07 C 51/573, C 07 C 27/26

(54) Verfahren zur Abtrennung von Jod und dessen Verbindungen aus den bei der Carbonylierung von Dimethylether, Methylacetat oder Methanol erhaltenen Carbonylierungsprodukten.

(30) Priorität: **30.09.85 DE 3534815**
**14.04.86 DE 3612504**

(43) Veröffentlichungstag der Anmeldung:
**08.04.87 Patentblatt 87/15**

(45) Bekanntmachung des Hinweises auf die Patenterteilung:
**22.06.88 Patentblatt 88/25**

(84) Benannte Vertragsstaaten:
**AT BE CH DE FR GB IT LI NL**

(56) Entgegenhaltungen:
**DE - A - 2 104 828**
**DE - A - 2 901 359**

(73) Patentinhaber: **HOECHST AKTIENGESELLSCHAFT,**
**Postfach 80 03 20, D-6230 Frankfurt am Main 80 (DE)**

(72) Erfinder: **Erpenbach, Heinz, Dr., Oberbuschweg 22,**
**D-5000 Köln (DE)**
Erfinder: **Gehrmann, Klaus, Dr.,**
**Geschwister-Scholl-Strasse 32, D-5042 Erftstadt (DE)**
Erfinder: **Hörstermann, Peter, Dr.,**
**Theodor-Heuss-Strasse 2, D-5042 Erftstadt (DE)**

## Beschreibung

Die Erfindung betrifft ein Verfahren zur Abtrennung von Jod und dessen Verbindungen aus den bei der Carbonylierung von Dimethylether und/oder Methylacetat und/oder Methanol in Gegenwart jodhaltiger Katalysatoren erhaltenen Carbonylierungsprodukten Essigsäure und/oder Acetanhydrid und/oder Ethylidendiacetat.

Die bei derartigen Carbonylierungsreaktionen, z. B. gemäss DE-A-28 36 084, entstehenden Reaktionsgemische werden üblicherweise destillativ aufgearbeitet. Dabei werden zunächst in einer ersten Trennstufe die nichtflüchtigen Katalysatorbestandteile von den flüchtigen Bestandteilen des Reaktionsgemisches abgetrennt. Von den flüchtigen Bestandteilen werden dann in einer Leichtsiederkolonne die niedrig siedenden Bestandteile Methyljodid und nichtumgesetztes Methylacetat und Dimethylether und ggf. Methanol über Kopf abdestilliert. In nachfolgenden Kolonnen werden dann zuerst Essigsäure, dann Essigsäureanhydrid und in einer letzten Kolonne ggf. Ethylidendiacetat über Kopf isoliert.

Die bei dieser Verfahrensweise erhaltenen Carbonylierungsprodukte enthalten als Verunreinigung aufgrund ihrer geringen Konzentration nur schwer spezifizierbare Jodverbindungen im ppm-Bereich und sind damit als Einsatzprodukte für technische Prozesse, die selbst wieder Edelmetallkatalysatoren benutzen, nur bedingt verwendbar.

Die DE-B-21 04 828 beschreibt ein Verfahren zur Entfernung geringer Halogenidverunreinigungen aus einer Essigsäure, welche vorzugsweise durch Reaktion eines Alkohols oder Olefins mit Kohlenmonoxid in Gegenwart eines Katalysatorsystems aus einer Edelmetall- und einer Halogenkomponente gewonnen wurde, durch Behandlung mit Kaliumpermanganat, Natriumpermanganat, Kaliumdichromat, Natriumdichromat, Chromtrioxid, Chromkaliumoxalat, Kaliumchlorchromat, Kaliumchlorat und/oder Kaliumchromat bei 16 bis 200° C. Hierbei wird eine weniger als 500 ppb Halogenid, insbesondere Jodid, enthaltende Essigsäure vor oder auch während der Destillation mit einer der genannten Metallverbindungen in einer Menge bis zu 1,0 Gew.-%, bezogen auf die Essigsäure, in Berührung gebracht, wobei man eine Abtrennung der Halogenidverbindung zwischen 90 und 98% erreicht. Zur Reinigung Acetanhydrid enthaltender Carbonylierungsprodukte ist dieses Verfahren weitaus weniger wirksam, da die genannten Oxydationsmittel auch mit Acetanhydrid reagieren und das Jodid daher nicht mehr binden können.

Die DE-A-22 56 510 beansprucht ein Verfahren zur Entfernung sehr geringer Mengen von Jod aus Essigsäure, bei dem in einem System aus zwei Destillationskolonnen die jodhaltigen Verbindungen durch Zusatz von Oxiden, Hydroxiden, Carbonaten, Bicarbonaten und Salzen schwacher organischer Säuren von Alkali- und Erdalkalimetallen sowie Gemischen der Alkali- oder Erdalkalimetallverbindungen mit Hypophosphoriger Säure je nach vorhandenem Jodgehalt bis zu einem Endgehalt von < 40 ppb bis < 5 ppb aus der eingesetzten Essigsäure entfernt werden können. Bei diesem Verfahren werden sowohl die Salze der Alkali- und Erdalkalimetalle als auch die hypophosphorige Säure in Form wässriger Lösungen eingesetzt. Das bedeutet, dass diese Reinigungsmethode zur Entfernung von verunreinigenden Jodverbindungen aus Essigsäureanhydrid nicht geeignet ist, da hierbei das Anhydrid einer Verseifung unterliegt.

Die DE-A-29 01 359 betrifft ein Verfahren zur Entfernung von Jod aus organischen Verbindungen, besonders aus Gemischen, die bei der oxidativen Acylierung von Olefinen mit Jod enthaltenden Katalysatoren erhalten wurden, z. B. Carbonsäureestern des Ethylen- oder Propylenglykols, wobei man die Jod enthaltenden organischen Verbindungen bei 50 bis 200° C mit einem Oxidationsmittel behandelt und das Reaktionsgemisch gleichzeitig oder anschliessend mit einem Adsorptionsmittel für Jod in Berührung bringt. Als Oxidationsmittel wird bevorzugt Sauerstoff oder Wasserstoffperoxid und als Adsorptionsmittel bevorzugt Aktivkohle verwendet. In den Beispielen wird als Oxidationsmittel Wasserstoffperoxid (20%ig) angegeben. Die Entfernung des Jods gelingt zu 92,4 bis 99,5%, wobei der Jodgehalt nur bis auf 40 ppm gesenkt wird, was den Reinheitsansprüchen bei weitem nicht genügt. Auch hier ist von Nachteil, dass durch den Einsatz von Wasserstoffperoxid dem System ebenfalls Wasser zugeführt wird.

Die DE-A-31 07 731 beansprucht ein Verfahren zur Abtrennung organischer Jodverbindungen von Carbonylierungsprodukten des Methanols, Methylacetats und Dimethylethers durch Flüssigphasenextraktion mit einem nichtaromatischen Kohlenwasserstoff. Hierbei werden gemäss den Beispielen die Jodgehalte nur bis auf höchstens 100 ppb Jod herabesetzt. Abgesehen von den zu hohen Jodwerten erfordert dieses Verfahren wegen der zusätzlichen Destillation des Extraktionsmittels einen wesentlich höheren Aufwand.

Nach der Lehre der DE-A-29 40 751 entfernt man als Verunreinigung vorhandene Jodverbindungen aus Carbonylierungsprodukten des Methylacetats durch Behandeln mit Cäsium-, Kalium- und/oder Natriumacetat, wobei man die entsprechenden Alkalimetalljodide erhält. Die Methyljodid-Abreicherung auf nur 2 ppm genügt nicht den Anforderungen an ein gereinigtes Carbonylierungsprodukt und dessen Weiterverarbeitung. Im übrigen wird durch die gaschromatographische Analyse auf Methyljodid der Gesamtjodgehalt im Carbonylierungsprodukt nicht vollständig erfasst, so dass eine Aussage über die wirkliche Jodabtrennung schwer nachvollziehbar ist.

Die vorliegende Erfindung beschreibt ein Verfahren, bei dem sich die Entfernung jodhaltiger Substanzen nicht nur auf Alkyl- und Aryljodide oder andere leicht destillierbare jodhaltige Verbindungen beschränkt, sondern bei dem es gelingt, Jod in jeder Art von gebundener wie auch elementarer Form von dem zu reinigenden Einsatzprodukt nahezu quantitativ abzutrennen. Als Beispiele ent-

fernbarer Jodverbindungen seien Butyljodid, Jodaceton, Acetyljodid, Jodwasserstoff sowie ggf. substituierte Ammonium- und Phosphoniumjodide genannt.

Im einzelnen ist das Verfahren der Erfindung nunmehr dadurch gekennzeichnet, dass man, zur Herabsetzung der Menge des die Carbonylierungsprodukte verunreinigenden Gesamtjods auf Gehalte unterhalb 20 ppb Jod, die Carbonylierungsprodukte bei Temperaturen von 20 bis 200° C mit Peressigsäure, Diacetylperoxid oder diese unter den Reaktionsbedingungen bildenden Verbindungen behandelt und destillativ abtrennt.

Das Verfahren der Erfindung ist weiterhin bevorzugt und wahlweise dadurch gekennzeichnet, dass man

a) Carbonylierungsprodukte mit weniger als 100 ppm Gesamtjod einsetzt,

b) die Behandlung der Carbonylierungsprodukte bei Temperaturen von 100° bis 140° C während 0,5 bis 120 min vornimmt,

c) die mit Peressigsäure, Diacetylperoxid oder diese unter den Reaktionsbedingungen bildenden Verbindungen behandelten Carbonylierungsprodukte fraktioniert destilliert.

Überraschenderweise werden gemäss Erfindung die in den bereits von Katalysator und den Leichtsiedern befreiten Carbonylierungsprodukten als Verunreinigungen noch enthaltenen, destillativ nur schwierig abtrennbaren Jodverbindungen oder elementares Jod durch die Behandlung mit Peressigsäure in destillativ leicht abtrennbare Jodverbindungen überführt, so dass ohne Verwendung von Adsorptionsmitteln nach erfolgter Reindestillation praktisch jodfreie Carbonylierungsprodukte erhalten werden.

Es ist ein besonderer Vorteil der Behandlung mit Peressigsäure, dass sie bei der Reaktion mit den Jodverbindungen in Gegenwart von Essigsäureanhydrid zu Essigsäure reagiert und so die Konzentration der im Gemisch der Carbonylierungsprodukte ohnehin enthaltenen Essigsäure nur geringfügig erhöht.

Die Peressigsäure wird in mindestens stöchiometrischen Mengen, berechnet auf den Gesamtjodgehalt in den Carbonylierungsprodukten, eingesetzt, doch ist sicherzustellen, dass das zur weiteren destillativen Aufarbeitung vorgesehene Gemisch der Carbonylierungsprodukte keine unumgesetzte Peressigsäure mehr enthält.

*Beispiel 1:*

1000 g eines Carbonylierungsproduktgemisches, das 80 Masse-% Essigsäureanhydrid, 20 Masse-% Essigsäure und 800 ppb Jod in Form nicht näher bekannter Jodverbindungen enthält, werden mit 30 g einer 10%igen wasserfreien Peressigsäurelösung in Eisessig versetzt und 60 min auf 120° C erhitzt. Die nachfolgende Fraktionierung ergibt reine Essigsäure und reines Essigsäureanhydrid mit Jodgehalten von < 10 ppb.

*Beispiel 2 (Vergleichsbeispiel)*

Beispiel 1 wird wiederholt mit dem Unterschied, dass keine Peressigsäurelösung zugesetzt wird.

Die bei der Fraktionierung erhaltene Essigsäure enthält 2800 ppb Jod und das erhaltene Essigsäureanhydrid enthält 300 ppb Jod.

*Beispiel 3:*

1000 g eines Carbonylierungsproduktgemisches, das 90 Masse-% Essigsäureanhydrid, 10 Masse-% Essigsäure und 1200 ppb Jod in Form nicht näher bekannter Jodverbindungen enthält, werden mit 20 g einer 10%igen wasserfreien Peressigsäurelösung in Eisessig versetzt und 15 min auf 132° C erhitzt. Bei der nachfolgenden Fraktionierung fallen reine Essigsäure und reines Essigsäureanhydrid mit Jodgehalten von < 10 ppb an.

*Beispiel 4 (Vergleichsbeispiel)*

Beispiel 3 wird wiederholt mit dem Unterschied, dass keine Peressigsäurelösung zugesetzt wird. Die bei der Fraktionierung erhaltene Essigsäure enthält 7000 ppb Jod und das erhaltene Essigsäureanhydrid enthält 400 ppb Jod.

*Beispiel 5:*

Zu 1000 g eines Carbonylierungsproduktgemisches, das 80 Masse-% Essigsäureanhydrid, 20 Masse-% Essigsäure und 1100 ppb Jod in Form nicht näher bekannter Jodverbindungen enthält, werden bei einer Temperatur von 132° C über einen Zeitraum von 15 min 20 g einer 10%igen wasserfreien Peressigsäurelösung in Eisessig kontinuierlich zudosiert. Die nachfolgende Fraktionierung ergibt Essigsäure und Essigsäureanhydrid mit Jodgehalten von < 10 ppb.

*Beispiel 6:*

In einer kontinuierlich betriebenen Versuchsanlage zur Herstellung von Essigsäureanhydrid durch Carbonylierung von Dimethylether und Methylacetat werden 100 Teile des nach der Abtrennung des Edelmetallkatalysators und der Leichtsieder Methyljodid und nichtumgesetztes Methylacetat erhaltenen Produktstromes aus 80 Masse-% Essigsäureanhydrid und 20 Masse-% Essigsäure, der einen Jodgehalt von 600 ppb besitzt, in einem Rohrreaktor bei 138° C mit einer mittleren Verweilzeit von 30 min kontinuierlich mit 3 Teilen einer 10%igen wasserfreien Lösung von Peressigsäure in Eisessig versetzt. Die nachfolgende Fraktionierung ergibt Essigsäure und Essigsäureanhydrid mit Jodgehalten von < 10 ppb.

*Beispiel 7:*

Beispiel 6 wird wiederholt mit dem Unterschied, dass die Peressigsäurelösung bei einer Temperatur von 138° C direkt in den Sumpf der Leichtsiederkolonne, in der das Methyljodid und nichtumgesetztes Methylacetat quantitativ über Kopf abgetrennt werden, eindosiert wird, wodurch sich eine mittlere Verweilzeit von 20 min ergibt. Bei der nachfolgenden Fraktionierung des Sumpfproduktes werden Essigsäure und Essigsäureanhydrid mit Jodgehalten von < 10 ppb erhalten.

*Beispiel 8 (Vergleichsbeispiel)*

Beispiel 7 wird wiederholt ohne Zusatz von Peressigsäurelösung zum Sumpf der Leichtsiederkolonne. Die nach der Fraktionierung erhaltene Essigsäure enthält 2400 ppb Jod und das erhaltene Essigsäureanhydrid enthält 150 ppb Jod.

## Patentansprüche

1. Verfahren zur Abtrennung von Jod und dessen Verbindungen aus den bei der Carbonylierung von Dimethylether, Methylacetat oder Methanol in Gegenwart jodhaltiger Katalysatoren erhaltenen Carbonylierungsprodukten Essigsäure, Acetanhydrid oder Ethylidendiacetat, dadurch gekennzeichnet, dass man, zur Herabsetzung der Menge des die Carbonylierungsprodukte verunreinigenden Gesamtjods auf Gehalte unterhalb 20 ppb Jod, die Carbonylierungsprodukte bei Temperaturen von 20° bis 200° C mit Peressigsäure, Diacetylperoxid oder diese unter den Reaktionsbedingungen bildenden Verbindungen behandelt und destillativ abtrennt.

2. Verfahren nach Anspruch 1, dadurch gekennzeichnet, dass man Carbonylierungsprodukte mit weniger als 100 ppm Gesamtjod einsetzt.

3. Verfahren nach mindestens einem der vorhergehenden Ansprüche, dadurch gekennzeichnet, dass man die Behandlung der Carbonylierungsprodukte bei Temperaturen von 100° bis 140° C während 0,5 bis 120 min vornimmt.

4. Verfahren nach mindestens einem der vorhergehenden Ansprüche, dadurch gekennzeichnet, dass man die mit Peressigsäure, Diacetylperoxid oder diese unter den Reaktionsbedingungen bildenden Verbindungen behandelten Carbonylierungsprodukte fraktioniert destilliert.

## Claims

1. Process for separating iodine and its compounds from the carbonylation products acetic acid, acetic anhydride or ethylidene diacetate obtained on subjecting dimethylether, methyl acetate or methanol to a carbonylation reaction in the presence of catalysts containing iodine, wherein in order to reduce the quantity of total iodine contaminating the carbonylation products to less than 20 ppb, the carbonylation products are treated at 20-200° C with peracetic acid, diacetyl peroxide or compounds yielding them under the reaction conditions, and separated distillatively.

2. Process as claimed in claim 1, wherein carbonylation products containing less than 100 ppm total iodine are used.

3. Process as claimed in at least one of the preceding claims, wherein the carbonylation products are treated at 100-140° C over a period of 0.5-120 minutes.

4. Process as claimed in at least one of the preceding claims, wherein the carbonylation products treated with peracetic acid, diacetyl peroxide or compounds yielding them under the reaction conditions are subjected to fractional distillation.

## Revendications

1. Procédé de séparation de l'iode et de ses composés des produits de carbonylation, acide acétique, anhydride acétique ou diacétate d'éthylidène, obtenus dans la carbonylation de l'éther diméthylique, de l'acétate de méthyle ou du méthanol en présence de catalyseurs contenant de l'iode, caractérisé en ce que, pour réduire la quantité d'iode total contaminant les produits de carbonylation à des teneurs de moins de 20 ppb, on traite les produits de carbonylation à des températures de 20-200° C par l'acide peracétique, le peroxyde de diacétyle ou des composés formant ces derniers dans les conditions réactionnelles, et on les sépare par distillation.

2. Procédé selon la revendication 1, caractérisé en ce que l'on utilise des produits de carbonylation d'une teneur en iode total inférieure à 100 ppm.

3. Procédé selon au moins l'une des revendications précédentes, caractérisé en ce que l'on traite les produits de carbonylation pendant 0,5-120 minutes à des températures de 100-140° C.

4. Procédé selon au moins l'une des revendications précédentes, caractérisé en ce que l'on soumet à une distillation fractionnée les produits de carbonylation traités par l'acide peracétique, le peroxyde de diacétyle ou des composés les formant dans les conditions réactionnelles.